(19) Europäisches Patentamt
European Patent Office
Office européen des brevets

(11) EP 0 933 358 B1

(12) # FASCICULE DE BREVET EUROPEEN

(45) Date de publication et mention de la délivrance du brevet:
**25.07.2001 Bulletin 2001/30**

(51) Int Cl.[7]: **C07C 319/26**, C07C 319/24, C07C 321/14

(21) Numéro de dépôt: **99400111.3**

(22) Date de dépôt: **19.01.1999**

(54) **Production de polysulfures organiques stabilisés et désodorisés**

Herstellung von stabilisierten und deodorierten organischen Polysulfiden

Production of stabilized and deodorized organic polysulfides

(84) Etats contractants désignés:
**AT BE CH DE ES FR GB GR IE IT LI NL PT SE**

(30) Priorité: **03.02.1998 FR 9801225**

(43) Date de publication de la demande:
**04.08.1999 Bulletin 1999/31**

(73) Titulaire: **Atofina**
**92800 Puteaux (FR)**

(72) Inventeurs:
- **Devaux, Jean-François**
  **64110 Jurançon (FR)**
- **Fremy, Georges**
  **64150 Os-Marsillon (FR)**
- **Labat, Yves**
  **33110 Le Bouscat (FR)**

(74) Mandataire: **Leboulenger, Jean et al**
**Atofina**
**D.C.R.D./D.P.I.**
**4, Cours Michelet**
**La Défense 10**
**92091 Paris la Défense Cedex (FR)**

(56) Documents cités:
**EP-A- 0 337 837**
**FR-A- 2 607 496**
**US-A- 3 595 820**
**US-A- 5 218 147**
**US-A- 5 403 961**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne le domaine des polysulfures organiques et a plus particulièrement pour objet la fabrication de polysulfures organiques stabilisés et désodorisés.

Les polysulfures organiques qu'on peut représenter par la formule générale $RS_nR'$ dans laquelle n est un entier allant de 2 à 15 et R et R' sont des radicaux hydrocarbyle éventuellement substitués, sont utilisés dans de nombreuses applications telles que, par exemple, la présulfuration des catalyseurs d'hydroraffinage et l'additivation d'élastomères. Ce sont aussi d'excellents additifs extrême-pression et anti-usure pour lubrifiants ; ils entrent donc dans la composition de formulations lubrifiantes pour boîtes de vitesse ou pour l'usinage des métaux. Pour la plupart des applications, on requiert un polysulfure inodore, exempt de précipité, clair et stable dans le temps.

Cependant, les polysulfures organiques sont généralement préparés par réaction d'un mercaptan sur le soufre élémentaire en présence d'un catalyseur basique ; ils peuvent aussi être préparés à partir d'une oléfine, de soufre et/ou d'hydrogène sulfuré, éventuellement en présence d'un catalyseur basique. Les produits ainsi préparés contiennent ou libèrent de l'hydrogène sulfuré et/ou des mercaptans dont l'élimination est difficile à mettre en oeuvre.

L'hydrogène sulfuré est toxique et, avec les mercaptans, à l'origine d'odeurs désagréables. D'autre part, en la présence éventuelle de restes catalytiques, le mercaptan et l'hydrogène sulfuré résiduels confèrent au produit fini une instabilité qui se traduit par une certaine turbidité ou l'apparition de précipités au cours du temps.

Afin de désodoriser et stabiliser les polysulfures organiques, il est indispensable d'éliminer les résidus de mercaptans et d'hydrogène sulfuré par un traitement approprié. Divers procédés pour résoudre ce problème ont déjà été décrits:

- lavage du polysulfure brut avec une solution d'acide et/ou de base (brevet US 5 155 275);
- lavage du polysulfure brut avec une solution oxydante en présence d'une quantité catalytique de base (brevet US 5 206 439);
- lavage du polysulfure brut avec une solution alcoolique ou aqueuse d'un sel métallique (brevet US 5 403 961);
- traitement du polysulfure brut par un acide et distillation subséquente (brevet US 5 530 163);
- traitement du polysulfure brut par un oxyde d'alcène en présence d'une quantité catalytique de base (brevets JP 58-140063 et US 5 218 147).

Ces procédés ne sont pas entièrement satisfaisants. Les procédés de lavage nécessitent l'ajout de solvants ou de solutions aqueuses qui sont difficilement séparables du polysulfure en fin de traitement, à moins de mettre en oeuvre des moyens de séparation long et coûteux. Le procédé avec une distillation finale est coûteux en énergie et ne s'applique qu'aux polysulfures légers. Les oxydes d'alcène sont toxiques et cancérogènes.

Certaines méthodes ne permettent pas, en un seul traitement, d'amener le taux de soufre mercaptan résiduel à une valeur suffisamment faible pour stabiliser le polysulfure; la nécessité de procéder à plusieurs traitements successifs en fait des méthodes longues et coûteuses.

Il a maintenant été trouvé un procédé de stabilisation des polysulfures et de réduction du taux de mercaptan résiduel, qui est rapide, utilise des réactifs peu toxiques et ne nécessite pas le recours à un solvant ou à un mélange de phase, ni à une distillation.

Le procédé selon l'invention pour la préparation d'un polysulfure organique stabilisé et désodorisé consiste à traiter un polysulfure organique brut par un carbonate d'alkylène en présence d'un catalyseur basique et, éventuellement, d'un donneur de soufre.

La présente invention va maintenant être décrite de façon détaillée.

### a- Définition du polysulfure de l'invention

Le polysulfure organique de la présente invention répond à la formule générale $RS_nR'$ dans laquelle n est un nombre allant de 2 à 15 et les symboles R et R', identiques ou différents, désignent généralement des radicaux hydrocarbyle ayant de 1 à 20 atomes de carbone et pouvant éventuellement présenter une ou plusieurs insaturations. Ces radicaux sont le plus souvent des radicaux alkyle, cycloalkyle ou aryle ; certains des atomes d'hydrogène des radicaux alkyle ou cycloalkyle peuvent être remplacés par des fonctions formyle ou acyle (-COR''), carboxy ou ester carboxylique (-COOR''), carbonitrile, azométhine (-CR''= NR'''), carboxamide (-CONR''R'''), nitro, hydroxy, alcoxy, amino (-NR''R'''), $-SiR''''_3$ ou par des groupements aryle ou cycloalkyle, les symboles R'' et R''', identiques ou différents, représentant chacun un atome d'hydrogène ou un radical alkyle et les trois substituants R'''', identiques ou différents, étant des radicaux alkyle ou alkoxy. De préférence, R et R' sont des radicaux alkyle, ramifiés ou non, ayant 4 à 12 atomes de carbone et n est un nombre allant de 3 à 6.

Le polysulfure organique peut également être le produit de la sulfurisation d'une oléfine, d'une polyoléfine, d'une oléfine polyinsaturée, d'une huile grasse, d'un ester gras ou d'un acide gras. Les oléfines sulfurisées comprennent

des produits faits par sulfurisation d'une oléfine (par exemple l'isobutylène) ou d'une polyoléfine (par exemple, le diisobutylène) avec du soufre, du chlorure de soufre, du dichlorure de soufre ou de l'hydrogène sulfuré ou une combinaison de ces produits, éventuellement en présence d'un catalyseur basique. Les huiles qui peuvent être sulfurisées sont des huiles naturelles ou synthétiques comprenant les huiles minérales, les huiles de lard, les esters carboxyliques dérivés d'alcools aliphatiques et d'acides gras ou d'acides carboxyliques aliphatiques, et les esters insaturés ou les glycérides. Les acides gras contiennent généralement de 8 à 30 atomes de carbone et sont, par exemple, l'acide palmitoléique, oléique, ricinoléique, linoléique, oléostéarique, ... Les esters d'acides gras sulfurisés peuvent être aussi préparés à partir de mélanges d'esters d'acides gras comme ceux obtenus à partir de graisses animales ou d'huiles végétales.

**b- Définition du polysulfure brut et mode de préparation (procédés déjà connus)**

Le polysulfure organique brut de la présente invention peut être préparé selon des procédé très divers. Une méthode de préparation connue dans l'art antérieur consiste à faire réagir un ou des mercaptans avec du soufre élémentaire en présence d'un catalyseur basique selon la réaction suivante :

$$RSH + R'SH + (n-1)S \rightarrow RS_nR' + H_2S$$

Des procédés de préparation de polysulfures organiques selon cette méthode sont décrits, par exemple dans les brevets US 2 237 625, US 3 022 351, US 3 038 013, US 3 392 201, US 4 564 709, US 5 206 439, US 5 530 163, FR 1 381 625, FR 1 553 249, FR 2 607 496, FR 2 635 775, EP 25944, EP 337837, WO 97/21649 et WO 97/21673.

Le polysulfure organique brut peut aussi être produit par sulfurisation d'une oléfine, polyoléfine, huile grasse, ester gras ou acide gras, selon des procédés décrits par exemple dans les brevets US 4 937 385, US 5 242 613, US 5 250 737, EP 201197, WO 92/3524 et WO 92/397.

A l'issue de la réaction qui conduit à la production du polysulfure brut, l'hydrogène sulfuré contenu dans le produit brut peut être en partie éliminé par dégazage, notamment soufflage d'un gaz inerte (avantageusement azote, air ou méthane, ...), par évaporation sous vide ou par toute autre méthode connue dans l'art.

c- Traitement selon l'invention du polysulfure brut

Conformément à la présente invention, le polysulfure brut est traité par un carbonate d'alkylène en présence d'un catalyseur basique et, éventuellement, d'un donneur de soufre.

Le carbonate d'alkylène utilisé dans la présente invention peut être le carbonate d'éthylène, le carbonate de propylène, le carbonate de butylène ou le carbonate de triméthylène et plus généralement tout composé de formule:

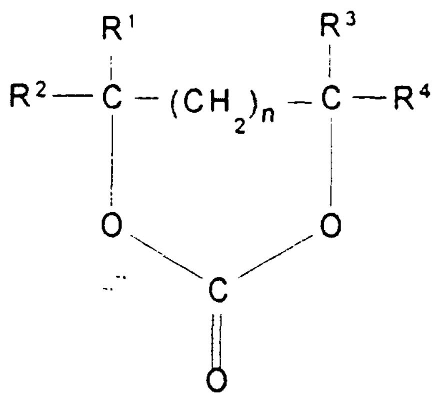

dans laquelle n est égal à 0, 1 ou 2 et les symboles $R^1$, $R^2$, $R^3$ et $R^4$, identiques ou différents, représentent chacun un atome d'hydrogène ou un radical alkyle. Le carbonate d'alkylène préféré est le carbonate d'éthylène, réactif peu onéreux et peu toxique, sa $LD_{50}$(oral, rat) étant égale à 10,4 g/kg.

La quantité de carbonate d'alkylène à utiliser dépend de la quantité de mercaptan(s) et d'hydrogène sulfuré dans le polysulfure brut. Généralement, on utilisera un ratio massique de carbonate d'alkylène par rapport au polysulfure brut allant de 0,001:1 à 0,2:1 et, de préférence, 0,005:1 à 0,1:1.

Avantageusement, si la quantité de mercaptan(s) et d'$H_2S$ restant dans le polysulfure brut est connue, on

utilisera un ratio molaire de carbonate d'alkylène par rapport à la somme des quantités molaires de mercaptan(s) et d'$H_2S$ compris entre 0,5:1 et 10:1, de préférence entre 0,9:1 et 2,5:1.

Comme exemples de catalyseurs basiques utilisables dans le procédé selon l'invention, on peut mentionner:

- des amines (comme la triéthylamine ou toute autre amine primaire, secondaire ou tertiaire), des hydroxydes d'ammonium, des alcanolamines,
- des mercaptides ou alcoolates tels que $R^5SNa$, $R^5ONa$, le phénoxyde de calcium ou de baryum, les composés $R^5O(CH_2CH_2O)_mNa$, $R^5S(CH_2CH_2O)_mNa$ ou un catalyseur constitué par la combinaison d'un mercaptan et d'un oxyde d'alcène avec une base alcaline (voir le brevet FR 2 607 496 dont le contenu est incoporé ici par référence), $R^5$ désignant un radical alkyle ou cycloalkyle et m un entier allant de 1 à 15,
- des hydroxydes métalliques tels que LiOH, NaOH et KOH,
- des oxydes métalliques ou des sels métalliques tels que $Na_2O$, $K_2O$, MgO, $NaHCO_3$, $Na_2CO_3$ et $CaCO_3$), ces composés pouvant être utilisés tels quels ou fixés sur un support (par exemple une alumine ou une silice),
- des zéolithes ou des hydrotalcites,
- des alumines, titanates, silices ou mélanges de ces composés, éventuellement modifiés par des alcalis ou des bases alcalino-terreuses,
- des résines basiques échangeuses d'anions telles que, par exemple, des résines à base de copolymère styrène-divinylbenzène fonctionnalisées par des groupements amine primaire, secondaire ou tertiaire, ammonium quaternaire, guanidine, amidine ou une combinaison de ces groupements.

Le catalyseur peut être employé tel quel ou en présence d'un solvant comme l'eau, un alcool, le disulfure de carbone ou tout autre solvant.

Le catalyseur basique de la présente invention peut déjà être contenu dans le polysulfure brut. Ceci est le cas notamment lorsque la réaction de synthèse du polysulfure brut utilise un catalyseur basique qui n'est pas séparé du polysulfure brut. Le catalyseur basique de la présente invention peut aussi être ajouté au polysulfure brut.

La quantité de catalyseur basique peut varier dans de larges limites. Généralement le catalyseur est utilisé en une quantité allant de 0,001 % à 50 % par rapport au poids du polysulfure brut, et de préférence 0,05 % à 25 %.

De préférence, on utilise comme catalyseur basique la combinaison d'un mercaptan et d'un oxyde d'alcène avec une base alcaline, ou une résine échangeuse d'anions. Dans le premier cas, la quantité de catalyseur est avantageusement de 0,001 à 10 % (de préférence 0,05 à 2 %) par rapport au poids de polysulfure brut. Lorsqu'on utilise une résine échangeuse d'anions, cette quantité est avantageusement de 0,1 à 50 % (de préférence 1 à 25 %) par rapport au poids du polysulfure brut.

Le procédé selon l'invention peut être réalisé dans n'importe quel équipement adapté. Celui-ci est avantageusement l'équipement utilisé pour la préparation du polysulfure brut. Après addition du carbonate d'alkylène et éventuellement du catalyseur basique sur le polysulfure brut (ou vice et versa), le mélange est agité à une température de 15 à 150°C (de préférence 50 à 120°C) pendant une durée de 2 minutes à 10 heures (de préférence 5 minutes à 5 heures).

Dans certains cas (notamment pour certains trisulfures tels le trisulfure de di-t-dodécyle ou le trisulfure de di-t-nonyle) et si le carbonate d'alkylène seul ne permet pas de diminuer suffisamment rapidement le taux de mercaptan résiduel, il peut être avantageux d'utiliser, en synergie avec le carbonate d'alkylène, une certaine quantité d'un donneur de soufre.

Le donneur de soufre peut être le soufre (sous n'importe qu'elle forme: liquide, en bille, en poudre,...) ou un polysulfure organique possédant 4 atomes de soufre ou plus par molécule. Les donneurs de soufre préférés sont le soufre, le pentasulfure de di-t-dodécyle et le pentasulfure de di-t-nonyle.

Si le donneur de soufre utilisé est le soufre, on utilisera généralement un ratio massique de donneur de soufre par rapport au polysulfure brut de 0:1 à 0,2:1 (de préférence 0:1 à 0,1:1). Si le donneur de soufre utilisé est un polysulfure organique, on utilisera généralement un ratio massique de donneur de soufre par rapport au polysulfure brut de 0:1 à 1:1 (de préférence de 0:1 à 0,2:1).

L'addition d'un donneur de soufre peut précéder celles du catalyseur et du carbonate d'alkylène, être simultanée ou bien être postérieure. L'addition simultanée de donneur de soufre et de carbonate d'alkylène est préférée. Le mélange est agité à une température de 15 à 150°C (de préférence 50 à 120°C) pendant une durée de 2 minutes à 10 heures (de préférence 5 minutes à 5 heures).

Le produit fini peut alors être purifié si nécessaire. Ceci peut être réalisé selon les méthodes conventionnelles de séparation telles une distillation ou une filtration.

Le procédé de cette invention peut aussi être adapté à une production continue.

Le procédé de cette invention tel qu'il a été décrit a pour effet de diminuer le taux de mercaptan résiduel et d'$H_2S$ à un niveau suffisamment faible pour que le polysulfure soit désodorisé et stabilisé.

Les exemples suivants illustrent l'invention Sauf indication contraire, les pourcentages indiqués sont des

pourcentages massiques.

**EXEMPLE 1 : Préparation du polysulfure de di-t-dodécyle ayant en moyenne 5 atomes de soufre - Finition au carbonate d'éthylène par catalyse homogène**

Dans un réacteur en acier inoxydable de 28 litres relié à la torche, on introduit 8,08 kg de t-dodécyl mercaptan et 60,6 g d'un catalyseur liquide obtenu par réaction de t-dodécyl mercaptan, d'oxyde d'éthylène et de soude selon le procédé décrit dans le brevet FR 2 607 496. Dans le mélange chauffé à 90°C et agité de façon continue, on introduit sur une durée d'une heure, 2,5 kg de soufre solide. Un fort dégagement gazeux d'$H_2S$ se produit et après 30 minutes, un fort courant de méthane (200 l/h) est insufflé dans le mélange pendant 1 heure 30, afin d'éliminer la majeure partie de l'$H_2S$ résiduel.

A l'issue de cette opération, le polysulfure brut obtenu contient 0,25 % de soufre mercaptan (déterminé par titration potentiométrique utilisant $AgNO_3$) ce qui correspond à 98 % de conversion du mercaptan initial.

Dans le même réacteur, on ajoute en une fois à 90°C 80 g de carbonate d'éthylène (correspondant à 1,2 équivalent molaire par rapport au mercaptan résiduel). Il se produit un dégagement gazeux significatif. Après 1 h 30 d'agitation à 90°C, le mélange est refroidi et filtré pour donner 9,9 kg de produit fini (> 99 % de rendement). L'analyse du produit fini montre une teneur en soufre mercaptan inférieure à 10 ppm. Le produit est stable et ne contient pas d'$H_2S$ et aucun précipité n'apparaît après 3 mois.

**EXEMPLE 2 : Préparation du polysulfure de di-t-dodécyle ayant en moyenne 5 atomes de soufre - Finition au carbonate d'éthylène par catalyse hétérogène.**

Dans un réacteur en acier inoxydable de 28 litres relié à la torche, un mélange de 8,08 kg de t-dodécyl mercaptan et 808 g de résine échangeuse d'ions Amberlyst® A21 (commercialisée par Röhm et Haas) est porté à 90°C sous agitation. 2,56 kg de soufre en billes sont alors introduits sur une durée d'une heure. Un fort dégagement gazeux d'$H_2S$ se produit et est éliminé à la torche. Après 30 minutes, un courant de méthane (200 l/h) est insufflé dans le mélange pendant 2 heure 30, pour éliminer la majeure partie de l'$H_2S$ résiduel.

A l'issue de cette opération, le polysulfure brut obtenu contient 0,32 % de soufre mercaptan ce qui correspond à une conversion de 97,6 % du mercaptan introduit.

Un échantillon de 50 g de ce polysulfure brut prélevé sans catalyseur est introduit dans un tricol de 100 ml muni d'un réfrigérant. On y ajoute 5 g de résine Amberlyst® A21 et 0,52 g de carbonate d'éthylène (CE) à 60°C. Après deux heures d'agitation à cette température, le taux de soufre mercaptan est inférieur à 10 ppm et le produit fini ne contient pas d'$H_2S$. Le produit est stable au cours du temps (essai 2 du tableau 1).

**EXEMPLE 3 : Comparatif**

Le polysulfure brut (50g) décrit à l'exemple 2 est chauffé à 90°C en présence de résine A21 sans carbonate d'éthylène. Après 2 heures, le taux de soufre mercaptan résiduel est de 1200 ppm et n'est pas stable (essai 3 du tableau 1).

Le polysulfure brut (50 g) décrit à l'exemple 2 est chauffé à 90°C sans résine en présence de 0,52 g de carbonate d'éthylène. Après 2 heures à 60 ou 90°C, le taux de mercaptan résiduel est toujours de 3200 ppm (essai 4 du tableau 1).

TABLEAU 1

| **Finition du polysulfure de di-t-dodécyle** (taux de soufre mercaptan avant réaction : 3200 ppm) | | | | | | |
|---|---|---|---|---|---|---|
| **Essai** | **Catalyseur résine** | **Réactif** | **Ratio molaire de réactif par rapport au mercaptan résiduel** | **Durée** | **Température** | **Taux de soufre mercaptan après la réaction** |
| 2 | A21 | CE | 1,5 | 2 h | 60°C | 10 ppm |
| 3 | A21 | aucun | 0 | 2 h | 90°C | 1200 ppm |
| 4 | aucun | CE | 1,5 | 2 h | 90°C | 3200 ppm |

**EXEMPLE 4 : Préparation du trisulfure de di-t-butyle**

Dans un réacteur en verre de 500 ml muni d'un réfrigérant à 0°C, on porte à 65°C un mélange de 135g de t-butyl mercaptan (TBM) et 1,69g du catalyseur liquide décrit à l'exemple 1. On introduit alors, sur une durée de 35 minutes et sous une agitation vigoureuse, 43,5 g de soufre solide. L'agitation est poursuivie à cette température pendant 90 minutes, puis la température est portée à 100°C et de l'azote est insufflé dans le mélange réactionnel avec une agitation vigoureuse pendant 120 minutes.

A ce stade, le taux de soufre mercaptan résiduel dans le polysulfure brut est de 1,27 %, ce qui correspond à une teneur massique en TBM de 3,5 %.

Le polysulfure brut est alors traité par 7,7 g de carbonate d'éthylène à 90°C. Il se produit un fort dégagement gazeux qui cesse au bout de 30 minutes. De l'azote est alors insufflé dans le mélange avec une agitation vigoureuse. Après 2 heures à 90°C, le produit est filtré. On obtient un liquide limpide, quasiment inodore et stable dans le temps, dont le taux de soufre mercaptan résiduel est inférieur à 15 ppm.

**EXEMPLE 5 : Comparatif**

Le polysulfure brut obtenu dans l'exemple 4 est chauffé pendant 2 h 30 à 90°C dans les mêmes conditions que dans l'exemple 4 avec soufflage d'azote mais sans carbonate d'éthylène. Le taux de soufre mercaptan reste élevé (2600 ppm) et le produit obtenu présente une odeur fortement nauséabonde.

**EXEMPLE 6 : Préparation du trisulfure de di-t-dodécyle**

Dans un réacteur en verre de 1 litre, on introduit 404 g de t-dodécyl mercaptan et 5 g du catalyseur liquide décrit dans l'exemple 1. Le mélange est chauffé à 110°C et agité de façon continue. On introduit alors, sur une durée de 15 minutes, 60,8 g de soufre en poudre. Un fort dégagement gazeux d'$H_2S$ se produit. Après 20 minutes, un fort courant d'azote est insufflé dans le mélange pendant 1 heure, afin d'éliminer la majeure partie de l'$H_2S$.

A l'issue de cette opération, le polysulfure brut obtenu contient 1,51 % de soufre mercaptan (déterminé par titration potentiométrique utilisant $AgNO_3$), correspondant à 90 % de conversion du mercaptan initial.

Dans le même réacteur, on ajoute en une fois à 110°C 19,5 g de carbonate d'éthylène (correspondant à 1,08 équivalent molaire par rapport au mercaptan résiduel) mélangé à 9,6 g de soufre en poudre. Il se produit un dégagement gazeux significatif. Après 1 h 30 d'agitation à 110°C, le mélange est refroidi et filtré. L'analyse du produit fini montre une teneur en soufre mercaptan inférieure à 9 ppm. Le produit est stable, ne contient pas d'$H_2S$ et aucun précipité n'apparaît après 3 mois.

**EXEMPLE 7 : Comparatif**

Le polysulfure brut de l'exemple précédent est traité à 110°C par 27,5 g de carbonate d'éthylène (correspondant à 1,5 équivalents molaires par rapport au mercaptan résiduel) sans ajout de donneur de soufre. Il se produit un dégagement gazeux très faible.

Après 10 heures d'agitation à cette température avec un bon stripping d'azote, la teneur en soufre mercaptan résiduel est encore supérieure à 0,5 % et le produit n'est pas stable.

## Revendications

1. Procédé de préparation d'un polysulfure organique stabilisé et désodorisé, caractérisé en ce qu'il consiste à traiter par un carbonate d'alkylène en présence d'un catalyseur basique, un polysulfure organique brut choisi parmi les produits de formule $RS_nR'$ dans laquelle n est un nombre allant de 2 a 15 et les symboles R et R', identiques ou différents, désignent des radicaux hydrocarbyle ayant de 1 à 20 atomes de carbone et pouvant présenter une ou plusieurs insaturations ou parmi les produits de sulfurisation d'une oléfine, d'une polyoléfine, d'une oléfine polyinsaturée, d'une huile grasse, d'un ester gras ou d'un acide gras, ce traitement étant effectué en outre en pésence d'un donneur de soufre dans le cas du trisulfure de dist-dodécyle ou de di-t-nonyle.

2. Procédé selon la revendication 1 dans lequel on utilise un carbonate d'alkylène de formule:

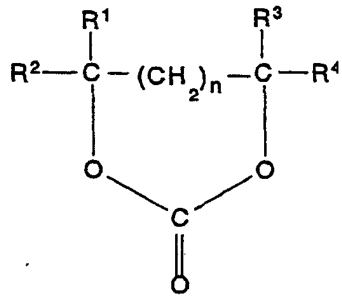

dans laquelle n est égal à 0, 1 ou 2 et les symboles $R^1$, $R^2$, $R^3$ et $R^4$, identiques ou différents, représentent chacun un atome d'hydrogène ou un radical alkyle.

3. Procédé selon la revendication 1 dans lequel le carbonate d'alkylène est le carbonate d'éthylène.

4. Procédé selon l'une des revendications 1 à 3 dans lequel le ratio massique du carbonate d'alkylène au polysulfure brut est compris entre 0,001:1 et 0,2:1, de préférence entre 0,005:1 et 0,1:1.

5. Procédé selon l'une des revendications 1 à 3 dans lequel le rapport molaire du carbonate d'alkylène à la somme des quantités molaires de mercaptan(s) et d'hydrogène sulfuré présentes dans le polysulfure brut est compris entre 0,5:1 et 10:1, de préférence entre 0,9:1 et 2,5:1.

6. Procédé selon l'une des revendications 1 à 5 dans lequel le catalyseur basique est utilisé en une quantité allant de 0,001 à 50 % (de préférence 0,05 à 25 %) par rapport au poids de polysulfure brut.

7. Procédé selon l'une des revendications 1 à 6 dans lequel le catalyseur basique est constitué par la combinaison d'un mercaptan et d'un oxyde d'alcène avec une base alcaline.

8. Procédé selon la revendication 7 dans lequel le catalyseur est utilisé en une quantité allant de 0,001 à 10 % (de préférence 0,05 à 2 %) par rapport au poids de polysulfure brut.

9. Procédé selon l'une des revendications 1 à 6 dans lequel le catalyseur basique est une résine échangeuse d'anions.

10. Procédé selon la revendication 9 dans lequel le catalyseur est utilisé en une quantité allant de 0,1 à 50 % (de préférence 1 à 25 %) par rapport au poids de polysulfure brut.

11. Procédé selon l'une des revendications 1 à 10 dans lequel le traitement est effectué à une température allant d'environ 15 à 150°C, de préférence 50 à 120°C.

12. Procédé selon l'une des revendications 1 à 11 dans lequel la durée du traitement est comprise entre 2 minutes et 10 heures, de préférence entre 5 minutes et 5 heures.

13. Procédé selon l'une des revendications 1 à 12 dans lequel, comme donneur de soufre, on utilise le soufre ou un polysulfure organique possédant au moins 4 atomes de soufre par molécule.

14. Procédé selon la revendication 13 dans lequel le donneur de soufre est le soufre utilisé dans un ratio massique du soufre au polysulfure brut allant de 0:1 à 0,2:1, de préférence 0:1 à 0,1:1.

15. Procédé selon la revendication 13 dans lequel le donneur de soufre est un polysulfure organique, de préférence le pentasulfure de di-t-dodécyle ou de di-t-nonyle, utilisé dans un ratio massique donneur de soufre/polysulfure brut allant de 0:1 à 1:1, de préférence 0:1 à 0,2:1.

16. Application du procédé selon l'une des revendications 1 à 15 à un polysulfure brut $RS_nR'$ dans lequel les symboles R et R' désignent des radicaux alkyle ayant de 4 à 12 atomes de carbone et n est un nombre allant de 3 à 6.

17. Application du procédé selon l'une des revendications 3 à 12 à la préparation d'un polysulfure de tert-butyle, de tert-nonyle ou de tert-dodécyle, ou du trisulfure de tert-butyle.

18. Application du procédé selon l'une des revendications 13 à 15 à la préparation du trisulfure de tert-dodécyle.

## Claims

1. Process for preparing a stabilized and deodorized organic polysulphide, characterized in that it consists in treating with an alkylene carbonate in the presence of a basic catalyst, a raw organic polysulphide selected from the products of the formula $RS_nR'$ in which n is a number ranging from 2 to 15 and the symbols R and R', which may be identical or different, denote hydrocarbyl radicals having 1 to 20 carbon atoms and possibly having one or more unsaturations, or from the sulphurization products of an olefin, a polyolefin, a polyunsaturated olefin, a fatty oil, a fatty ester or a fatty acid, this treatment being further carried out in the presence of a sulphur donor in the case of di-t-dodecyl or di-t-nonyl trisulphide.

2. Process according to Claim 1, in which use is made of an alkylene carbonate of formula

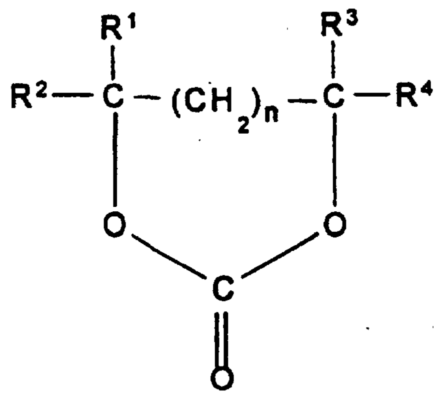

in which n is equal to 0, 1 or 2 and the symbols $R^1$, $R^2$, $R^3$ and $R^4$, which may be identical or different, each represent a hydrogen atom or an alkyl radical.

3. Process according to Claim 1, in which the alkylene carbonate is ethylene carbonate.

4. Process according to one of Claims 1 to 3, in which the mass ratio of the alkylene carbonate to the raw polysulphide is between 0.001:1 and 0.2:1, preferably between 0.005:1 and 0.1:1.

5. Process according to one of Claims 1 to 3, in which the molar ratio of the alkylene carbonate to the sum of the molar quantities of mercaptan(s) and hydrogen sulphide present in the raw polysulphide is between 0.5:1 and 10:1, preferably between 0.9:1 and 2.5:1.

6. Process according to one of Claims 1 to 5, in which the basic catalyst is used in an amount ranging from 0.001 to 50% (preferably 0.05 to 25%) relative to the weight of raw polysulphide.

7. Process according to one of Claims 1 to 6, in which the basic catalyst consists of the combination of a mercaptan and an alkene oxide with an alkaline base.

8. Process according to Claim 7, in which the catalyst is used in an amount ranging from 0.001 to 10% (preferably 0.05 to 2%) relative to the weight of raw polysulphide.

9. Process according to one of Claims 1 to 6, in which the basic catalyst is an anion-exchange resin.

10. Process according to Claim 9, in which the catalyst is used in an amount ranging from 0.1 to 50% (preferably 1 to 25%) relative to the weight of raw polysulphide.

**11.** Process according to one of Claims 1 to 10, in which the treatment is carried out at a temperature ranging from about 15 to 150°C, preferably 50 to 120°C.

**12.** Process according to one of Claims 1 to 11, in which the duration of the treatment is between 2 minutes and 10 hours, preferably between 5 minutes and 5 hours.

**13.** Process according to one of Claims 1 to 12, in which the sulphur donor used is sulphur or an organic polysulphide having at least 4 sulphur atoms per molecule.

**14.** Process according to Claim 13, in which the sulphur donor is sulphur used in a mass ratio of the sulphur to the raw polysulphide ranging from 0:1 to 0.2:1, preferably 0:1 to 0.1:1.

**15.** Process according to Claim 13, in which the sulphur donor is an organic polysulphide, preferably di-t-dodecyl or di-t-nonyl pentasulphide, used in a sulphur donor/raw polysulphide mass ratio ranging from 0:1 to 1:1, preferably 0:1 to 0.2:1.

**16.** Application of the process according to one of Claims 1 to 15 to a raw polysulphide $RS_nR'$ in which the symbols R and R' denote alkyl radicals having 4 to 12 carbon atoms and n is a number ranging from 3 to 6.

**17.** Application of the process according to one of Claims 3 to 12 to the preparation of tert-butyl, tert-nonyl or tert-dodecyl polysulphide, or tert-butyl trisulphide.

**18.** Application of the process according to one of Claims 13 to 15 to the preparation of tert-dodecyl trisulphide.

**Patentansprüche**

**1.** Verfahren zur Herstellung eines stabilisierten und desodorisierten organischen Polysulfids, dadurch gekennzeichnet, daß es darin besteht, mit einem Alkylencarbonat in Gegenwart eines basischen Katalysators ein rohes organisches Polysulfid zu behandeln, das aus den Produkten der Formel $RS_nR'$, in der n eine Zahl von 2 bis 15 ist und die gleichen oder verschiedenen Symbole R und R' Kohlenwasserstoffreste mit 1 bis 20 Kohlenstoffatomen bezeichnen, die eine oder mehrere ungesättigte Stellen aufweisen können oder aus den Produkten einer Sulfurierung eines Olefins, eines Polyolefins, eines mehrfach ungesättigten Olefins, eines Fettöls, eines Fettesters oder einer Fettsäure gewählt ist, wobei diese Behandlung im Fall des Di-t-dodecyl- oder Di-t-nonyltrisulfids außerdem in Gegenwart eines Schwefeldonators durchgeführt wird.

**2.** Verfahren nach Anspruch 1, in dem man ein Alkylencarbonat verwendet mit der Formel:

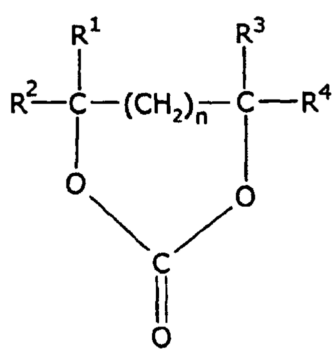

in der n gleich 0, 1 oder 2 ist und die gleichen oder verschiedenen Symbole $R^1$, $R^2$, $R^3$ und $R^4$ jeweils ein Wasserstoffatom oder einen Alkylrest bedeuten.

**3.** Verfahren nach Anspruch 1, in dem das Alkylencarbonat Ethylencarbonat ist.

**4.** Verfahren nach einem der Ansprüche 1 bis 3, in dem das Massenverhältnis des Alkylencarbonats zum rohen Polysulfid zwischen 0,001:1 und 0,2:1, vorzugsweise zwischen 0,005:1 und 0,1:1, liegt.

5. Verfahren nach einem der Ansprüche 1 bis 3, in dem das Molverhältnis des Alkylencarbonats zur Summe der Molmengen des/der in dem rohen Polysulfid vorliegenden Mercaptane/s und des Schwefelwasserstoffs zwischen 0,5:1 und 10:1, vorzugsweise zwischen 0,9:1 und 2,5:1, liegt.

6. Verfahren nach einem der Ansprüche 1 bis 5, in dem der basische Katalysator in einer Menge von 0,001 bis 50 % (vorzugsweise 0,05 bis 25 %) bezogen auf das Gewicht des rohen Polysulfids verwendet wird.

7. Verfahren nach einem der Ansprüche 1 bis 6, in dem der basische Katalysator aus der Kombination eines Mercaptans und eines Alkenoxids mit einer alkalischen Base besteht.

8. Verfahren nach Anspruch 7, in dem der Katalysator in einer Menge von 0,001 bis 10 % (vorzugsweise 0,05 bis 2 %), bezogen auf das Gewicht des rohen Polysulfids, verwendet wird.

9. Verfahren nach einem der Ansprüche 1 bis 6, in dem der basische Katalysator ein Anionenaustauscherharz ist.

10. Verfahren nach Anspruch 9, in dem der Katalysator in einer Menge von 0,1 bis 50 % (vorzugsweise 1 bis 25 %) bezogen auf das Gewicht des rohen Polysulfids verwendet wird.

11. Verfahren nach einem der Ansprüche 1 bis 10, in dem die Behandlung bei einer Temperatur von ungefähr 15 bis 150 °C, vorzugsweise 50 bis 120 °C, durchgeführt wird.

12. Verfahren nach einem der Ansprüche 1 bis 11, in dem die Behandlungsdauer zwischen 2 Minuten und 10 Stunden, vorzugsweise zwischen 5 Minuten und 5 Stunden, liegt.

13. Verfahren nach einem der Ansprüche 1 bis 12, in dem man als Schwefeldonator Schwefel oder ein organisches Polysulfid mit mindestens 4 Schwefelatomen pro Molekül verwendet.

14. Verfahren nach Anspruch 13, in dem der Schwefeldonator Schwefel ist, der in einem Massenverhältnis des Schwefels zum rohen Polysulfid von 0:1 bis 0,2:1, vorzugsweise von 0:1 bis 0,1:1, verwendet wird.

15. Verfahren nach Anspruch 13, in dem der Schwefeldonator ein organisches Polysulfid, vorzugsweise Di-t-dodecyl- oder Di-t-Nonylpentasulfid ist, das in einem Massenverhältnis Schwefeldonator/rohes Polysulfid von 0:1 bis 1:1, vorzugsweise 0:1 bis 0,2:1, verwendet wird.

16. Anwendung des Verfahrens nach einem der Ansprüche 1 bis 15 auf ein rohes Polysulfid $RS_nR'$, in dem die Symbole R und R' Alkylreste mit 4 bis 12 Kohlenstoffatomen bezeichnen und n eine Zahl von 3 bis 6 ist.

17. Anwendung des Verfahrens nach einem der Ansprüche 3 bis 12 zur Herstellung eines tert.-Butyl-, tert.-Nonyl- oder tert.-Dodecylpolysulfids oder eines tert.-Butyltrisulfids.

18. Anwendung des Verfahrens nach einem der Ansprüche 13 bis 15 zur Herstellung von tert.-Dodecyltrisulfid.